# EUROPEAN PATENT SPECIFICATION

(11) **EP 1 813 338 B1**
(45) Date of publication and mention of the grant of the patent: **12.10.2011**
(21) Application number: 05767233.9
(22) Date of filing: 28.07.2005
(51) Int. Cl.: B01D 39/14, A61L 9/01, B01D 46/00

(54) **USE OF AN AIR FILTER WITH GINKGO EXTRACT**
VERWENDUNG EINES LUFTFILTERS MIT GINKGOEXTRAKT
UTILISATION D'UN FILTRE A AIRE AVEC EXTRAIT DE GINKGO

(30) Priority: 29.07.2004 JP 2004222548
(43) Date of publication of application: 01.08.2007
(73) Proprietor: MITSUBISHI PAPER MILLS LIMITED, Tokyo 100-0005 (JP)
(72) Inventor: OKU, Yasuyuki, c/o MITSUBISHI PAPER MILLS LIMITED, Chiyoda-ku, Tokyo 100-0005 (JP); OGAMI, Katsushi, c/o MITSUBISHI PAPER MILLS LIMITED, Chiyoda-ku, Tokyo 100-0005 (JP)
(74) Representative: Albrecht, Thomas
(86) International application number: PCT/JP2005/013805
(87) International publication number: WO 2006/011541

(56) References cited:
- EP-A- 1 566 170
- WO-A-03/013285
- JP-A- 6 078 739
- JP-A- 8 266 261
- JP-A- 10 072 316
- JP-A- 61 103 447
- JP-A- 2000 005 531
- JP-A- 2001 269 518
- JP-A- 2002 128 680
- KR-A- 20020 070 598
- US-A- 5 686 082

## Description

The present invention relates to the use of an air filter comprising a breathable support with a ginkgo extract contained therein, said breathable support having a plane shape
for inactivating an allergen trapped on the air filter wherein the application is an air processing device.

### [Background art]

Recently, people who complain about allergy symptoms such as itching, a runny nose, tears, sneezing and coughing (asthma) have increased as living environment changed, and this has caused a huge social problem. As an allergy-inducing substance, attention has been focused on a protein allergen which is a plant-derived substance such as cedar pollen, Japanese cypress pollen and Solidago altissima pollen, and is an animal-derived substance such as tick feces, dead tick, and body hair of dogs or cats. It has become a problem how to remove these substances.

Many of allergies which have become problems in recent years are caused by inhalation of an allergen substance floating in air followed by an intense antigen-antibody reaction in the body. Therefore, it may be conjectured that the allergy symptoms may be alleviated by removing or trapping the allergen substance in the air in some ways. In such a background, a technology for trapping pollens and ticks (including derivatives thereof) by a dust removal filter is known in the art of filter, and the trapping accuracy tends to be increased. However, a technology for reducing or inactivating the trapped allergen itself by a filter is not still sufficient.

Allergens derived from organism such as ticks and pollens contain proteins as a major component. Accordingly, it is conjectured that an activity as an allergen may be deactivated by denaturalizing the allergen proteins.

As an air filter, there are disclosed a filter supporting (carrying) a tea catechin thereon (for example, see Patent Literature 1) or a filter supporting an enzyme thereon (for example, see Patent Literature 2). Also, there is disclosed a mask supporting an aromatic hydroxyl compound as an anti-allergen component thereon (for example, see Patent Literature 3), or the like, and it is expected to be used as a filter.

On the other hand, plant-derived extracts have been reported to have many physiologically active effects from a long time ago, and they are used in healthy foods or drinks. Among them, an extract extracted from ginkgoes has been reported to have various pharmacological effects. The ginkgo extract contains ginkgo-inherent substances such as various kinds of flavonol glycosides, ginkgolides and bilobalides. It is conjectured that these ginkgo- inherent substances show a specific physiologically active effect which is not seen in the other plant extracts.

To cite an example with respect to controlling an allergic reaction, there are disclosed a herbal composition and a treatment for an allergic reaction (for example, see Patent Literature 4), a composition having an antiinflammatory effect or anti-allergic effect, having less side-effect and capable of being taken over the long term (for example, see Patent Literature 5). There is also disclosed that an allergic reaction can be controlled by taking the ginkgo extract as a safe natural component. It is believed that a component contained in the ginkgo leaf extract can control an allergic reaction caused in the human body.
Each of them has declared an effect of inhibiting histamine overproduced in the body, which is a cause of inducing the allergic reaction, by a foreign substance (allergen) which has externally invaded the body but has not elucidated an effect on the allergen itself.

Additionally, although it has been reported that taking the ginkgo leaf extract as a food or medicine leads to various physiological activities, there is no report which refers to a physiological activity when the ginkgo leaf extract is supported onto a support such as non-woven fabric, and no report which provides an evidence of the physiological activity in such a case. That is, even the clue is not found for a report which refers to a direct allergen inhibiting effect or reducing effect on the allergen. Of course, there has so far been no example that the ginkgo extract is applied as an air filter having an allergen-reducing effect.
Incidentally, with the use for patients suffering from atopic dermatitis in mind, there is a proposal for a method of using a plant-extracted extract as a house-dust treating agent by spraying it from a spray to a carpet (for example, see Patent Literatures 6 and 7). Alternately, there is also a proposal for a smoking filter based on the use of breathing of a specific user (for example, see Patent Literature 8).
However, there has been no proposal for an application based on such a using method that not individually-controlled amount of ginkgo leaf extract component is dispersed continuously at all time in a place where the general public (of course, a group where people of various constitutions exist together) resides, for example, an air filter to be used as a member of the air processing device (for example, air cleaner).
[Patent Literature 1]
   Japanese Laid-Open Patent [Kokai] Publication No. 2000-5531
[Patent Literature 2]
   Japanese Laid-Open Patent [Kokai] Publication No. 2003-210919
[Patent Literature 3]
   Japanese Laid-Open Patent [Kokai] Publication No. 2003-79756
[Patent Literature 4]
   Published Japanese translation of a PCT application No. 2002-541115
[Patent Literature 5]
   Japanese Laid-Open Patent [Kokai] Publication No. 2000-14354
[Patent Literature 6]
   Japanese Laid-Open Patent [Kokai] Publication No. 2002-128680
[Patent Literature 7]
   Japanese Laid-Open Patent [Kokai] Publication No. 2002-128659
[Patent Literature 8]
   Japanese Laid-Open Patent [Kokai] Publication No. Hei 6-78739
JP 10072316 teaches an antibacterial composition comprising a ginkgo inner seed coat extract as an active ingredient. The problem solved by this invention is to provide a low-allergy, mildly-irritating and low-toxic antibacterial composition, without using bactericidal agents or preservative agents which might have toxicity and irritant property and might be allergen causing allergy thereby having potential to cause harm to the living body, in particular to human body, yet possessing antibacterial efficacy equivalent to or more than that in the case of using bactericidal agents or preservative agents for a long time (abstract, and [0009]). The inventors of JP 10072316 had studied preservatives having high safety as well as low-allergy property, mildly-irritating property and low-toxicity, and as a result, had found that extracts from ginkgo inner skin have high anti-bacterial activity, and that pharmaceuticals, foods, skin external preparations and cosmetics containing the extracts also have anti-bacterial activity without providing irritation or discomfort feeling to the living body, in particular, to the skin ([0010]).
That is, what is described in this document regarding the allergen is that the extracts from ginkgo inner skin are not allergens causing allergy to the skin or the living body.

### [Disclosure of the Invention]

### [Problems to be Solved by the Invention]

The present invention is to solve the above-mentioned problems, and aims to provide the use of an air filter supporting a ginkgo extract onto a breathable support and inactivating an allergen.

### [Means for Solving the Problems]

Namely, the present invention comprises the following constitutions:
(1) The present invention relates to the use of an air filter comprising a breathable support with a ginkgo extract contained therein, said breathable support having a plane shape , for inactivating an allergen trapped on the air filter wherein the application is an air processing device.
(2) According to another aspect, the present invention relates to the use according to item (1) above, wherein the air processing device is an air cleaner.
(3) According to another embodiment, the present invention relates to the use according to item (1) or (2) above, wherein the ginkgo extract is obtained by extraction of ginkgo leaves with a solvent selected from a group consisting of water, ethyl alcohol, acetone, ethyl ether, propylene glycol, butylene glycol, glycerin and acetic acid.
(4) According to another embodiment, the present invention relates to the use according to any one of items (1) to (3) above, wherein the breathable support is fabricated into a three-dimensional shape.
(5) According to another embodiment, the present invention relates to the use according to any one of items (1) to (4), wherein the amount of the ginkgo extract is 0.1 g/m² to 10.0 g/m² based on the area of the breathable support.
(6) According to another embodiment, the present invention relates to the use according to item (5), wherein the amount of the ginkgo extract is 0.5 g/m² to 5.0 g/m² based on the area of the breathable support.
(7) According to another embodiment, the present invention relates to the use according to any one of items (1) to (6), wherein the ginkgo extract contains flavonol glycosides and terpene lactones.
(8) According to another embodiment, the present invention relates to the use according to item (7), wherein the amount of the flavonol glycosides is 300% by weight to 1500% by weight based on the amount of the terpene lactones.
(9) According to another embodiment, the present invention relates to the use according to item (7), wherein the amount of the flavonol glycosides is 0.0004 g/m² to 0.15 g/m² based on the area of the breathable support.

### [Effect of the Invention]

The air filter used in the present invention is obtained by supporting a ginkgo extract onto a breathable support. The air filter used in the present invention is effective as an air filter because it is able to inactivate an allergen trapped on the filter. Furthermore, the air filter of the present invention also has an antiviral activity and an antibacterial activity.
The inactivation of an allergen (for example, allergens derived from ticks and cedar pollens) trapped on a filter containing a ginkgo extract (an allergen contacted with the filter) has had a practical value for the first time in the application that the general public uses by the present invention.

### [Best Mode for Carrying Out the Invention]

In the following, an air filter used in the present invention will be explained in detail.

In the present invention, a ginkgo extract refers to an extract extracted from ginkgo leaves, ginkgo seeds, ginkgo bark, ginkgo stem or the like. It is not particularly limited so long as it is able to denaturalize or decompose a protein which constitutes an allergen. As noted in the next, an extract extracted from ginkgo leaves (ginkgo leaf extract) may be particularly preferably used and there is no specific problem in using an extract from a portion other than leaves.

Especially, the ginkgo leaf extract contains many physiologically active substances which are not found in an extract extracted from the other natural products. As one example thereof, there may be mentioned three kinds of flavonol glycosides, quercetin, isorhamnetin and kaempferol, and terpene lactones such as ginkgolide and bilobalide, or the like.

It is known that these active substances have various effective functions such as improvement in cardiovascular function, improvement in blood flow promotion, antiaging, keeping the stomach healthy, keeping the eyes healthy and keeping the brain healthy. The ginkgo leaf extract contains about 20 kinds of flavonol glycosides. Among them, there are included as much as 6 kinds of specific biflavones which are contained only in the ginkgo leaf (ginkgetin, isoginkgetin, bilobetin, 5-methoxybilobetin amentoflavone, sciadopitysin or the like). They have a function to vasodilate the blood vessel (in particular, capillary vessel) and promote the blood circulation. They also have a water retention ability, a moisture retention ability and an ability to soften the skin, and prevent spots and freckles.

Also, among the terpene lactones, a ginkgolide, in particular, has an activity of inhibiting the action of a substance which induces an allergic reaction (platelet-activating factor (PAF)). Therefore, it is said that taking the ginkgolide serves in the prevention of or improvement in allergy symptoms. Furthermore, it is also said that the ginkgolide has an activity of inhibiting an active oxygen and the power of the activity is several hundreds of times than vitamin E.
Incidentally, terpene lactones contained in the ginkgo leaf extract include the diterpene ginkgolide and the sesquiterpene bilobalide. Furthermore, there are 4 types of ginkgolides A, B, C and J.

The function and mechanism of the anti-allergen effect of the air filter used in the present invention are completely different from those of the effect of preventing from the allergic reaction as mentioned above. There are still some not-clarified points in the allergen inactivation action. However, a filter comprising the ginkgo extract carried thereon is assumed not only to reduce an allergen on the filter by the potent complexed activity of these specific components but also to exhibit an antiviral activity or antibacterial activity.

A method for extracting the ginkgo extract is not particularly limited and the ginkgo extract can be obtained by a previously disclosed method. To cite one example, in the case where the ginkgo leaf extract is to be used in the present invention, it is preferred to extract the ginkgo extract from ginkgo leaves by using a solvent such as water, ethyl alcohol, acetone, ethyl ether, propylene glycol, butylene glycol, glycerin and acetic acid. The extraction operation is not particularly limited, and in the case of using the ginkgo leaves, for example, extraction may be carried out by using a solvent in an amount capable of effectively extracting the ginkgo leaf extract. Further, it is more preferred to carry out the extraction operation at a temperature of 10 to 80 °C for 1 to 24 hours.

For the ginkgo extract of the present invention, an extract liquid obtained as mentioned above may be used as- is, or optionally, a component obtained by purifying, concentrating and drying the extract liquid according to the conventional method may be used as a powder, or the one obtained by further diluting the extract liquid may be used.

Incidentally, when an extract obtained according to the above-mentioned extraction method and the like is analyzed by high performance liquid chromatography and the like, quality of the extract to be used can be confirmed. Furthermore, the above-mentioned extraction method can be applied as is to the analysis of the ginkgo extract components contained in the air filter used in the present invention. That is, by analyzing the liquid extracted from the filter by the extraction process, quality of the filter after the filter production can be confirmed, whereby a safe production become possible.

When an air filter having a constant quality is to be obtained by using the ginkgo extract, there was a problem that there are differences in the amounts of the respective components contained in the extract depending on the portions of the ginkgo (leaves, seeds, bark, stem or the like). With respect to leaves, in particular, it is known that the contents of the respective components dramatically vary depending on seasons, insolation to the tree or the like.

The present inventors have earnestly studied, and as a result, they have found that an air filter having a constant quality can be produced by keeping the relative ratio of the amount of a specific component contained in the ginkgo extract constant. This relative ratio of the amount of a specific component contained in the ginkgo extract is also reflected to the air filter. In the following, this will be explained in detail.

The ginkgo extract contains various components such as flavonol glycosides, terpene lactones, alkyl phenol and the like. Among them, the contained amount of the flavonol glycosides is preferably 300% by weight or more to 1500% by weight or less, more preferably 500% by weight or more, and further preferably 600% by weight or more based on the amount of the terpene lactones. When the contained amount of the flavonol glycosides exceeds 1500% by weight, it may be difficult to stably obtain the extract. In the case where the flavonol glycosides are contained in the above-mentioned range, some effects such as allergen reduction and the like by the air filter of the present invention can be accomplished constantly and continuously. The preferred content ratio of flavonol glycosides as mentioned above is applied to the air filter as is as a preferred content ratio in the air filter. Furthermore, the concentration of the above-mentioned component in the ginkgo leaf extract solution is preferably 0.01 to 5% by weight, more preferably 0.1 to 3% by weight based on the ginkgo leaf extract solution.

As a method for easily obtaining the ginkgo extract which fulfill the above-mentioned component ratio, there may be mentioned an extraction from ginkgo fresh leaves of spring according to the conventional method. (Hereinafter, this extract may be referred to as a ginkgo fresh leaf extract.) This is because that the ginkgo flesh leaf extract contains a relatively-high amount of flavonol glycosides, while it contains less amount of terpene lactones than those in the other seasons. In this regard, however, the above-mentioned component ratio is more important than the amounts of the respective components contained in the air filter in obtaining a constant effect.

The quality control due to the component ratio in the extract as mentioned above also serves in the effective utilization of a ginkgo extract other than the ginkgo fresh leaf extract (a ginkgo leaf extract in the turn of the year, of summer or of autumn, respectively, or a ginkgo extract from the portions other than leaves). For example, as in the case where the extraction method is changed in order to extract flavonol glycosides as much as possible, on the other hand, to extract terpene lactones as less as possible, an extraction solvent, extraction temperature, time or the like can be changed. In addition, if the extract in combination with a ginkgo fresh leaf extract and the like fulfils the above-mentioned component ratio, it may be preferably used in the present invention.
Incidentally, ginkgoes have various subspecies and components in an extract obtained from the subspecies also varies depending on the subspecies. However, the quality control due to the component ratio in the extract is the same as in the above.

The amount of the ginkgo extract to be contained in the air filter used in the present invention is preferably 0.1 g/m² or more to 10.0 g/m² or less, more preferably 0.5 g/m² or more to 5.0 g/m² or less based on the area of the filter. The amount of ginkgo extract within this range is preferred since some effects such as allergen reduction can be obtained to a satisfactory extend by containing this amount of ginkgo extract in the filter. If the amount of the ginkgo extract falls below such a lower limit, the effect of the present invention may not be obtained sufficiently. Also, if the amount of the ginkgo extract exceeds such an upper limit, some people sensitive to a smell may find the odor of the ginkgo extract itself uncomfortable although some effects such as allergen reduction can be obtained. In this regard, however, the ginkgo extract have a difference in the contents of the respective components depending on seasons and the like, as mentioned above. Therefore, as an indicator for the filter production and the quality confirmation, it is more effective and practical to depend on the content of the flavonol glycosides as mentioned below.

As an easy means to know whether the amount of the ginkgo leaf extract to be contained in the filter is preferred or not, there may be mentioned a method for measuring the content of the flavonol glycosides as mentioned above. If the content of the flavonol glycosides is preferably 0.0004 g/m² or more to 0.15 g/m² or less, more preferably 0.002 g/m² or more to 0.12 g/m² or less, the contained amount of the extract may be deemed to be in the above preferred range. Incidentally, with respect to the upper limit of the preferred contained amount of the extract, a sensory test according to the confirmation of the presence or absence of the order specific for the extract is easier than any analysis. More strictly speaking, when a little odor is felt, it is approved if one feels the odor comfortable and it is disapproved if one feels the odor uncomfortable. On the other hand, in the case of employing a method according to the measurement of the content, the contained amount of the extract is deemed as being in the preferred range if the content of the flavonol glycosides is 0.15 g/m² or less.
In addition, in a manufacturing field where extracts extracted from various kinds of plants are treated, to prevent from mistaking a ginkgo extract for the other materials, detecting a specific component contained in the ginkgo extract such as ginkgolide from the extract or filters comprising the same allows to confirm it as the ginkgo extract.

Next, a breathable support to be used in the present invention will be explained. In the present invention, a material for the breathable support to be used is not particularly limited, and any material may be used so long as it is compatible with a shape of a device or instrument to be used. Any shape such as a plane shape or a solid shape may be used.

A plane shape includes a sheet-like shape as one example. The support can be appropriately selected from sheet-like materials such as a fabric, a knitted good, a nonwoven fabric, paper, net, a breathable form, a metallic porous material and holed film. As one of preferred supports, there may be mentioned a nonwoven fabric in terms of inexpensive price, easiness of controlling the fiber blend, coating weight or thickness, and easiness of processing after opening the hole, etc. As a process for making a nonwoven fabric, at least one process selected from spun bond process, melt blown process, dry process (thermal bond process, resin bond process, needle punch process, spun lace process, stitch bond process), and wet process, can be used. Optionally, it is possible to use a plural number of these processes in combination.

As a method for carrying (supporting) a ginkgo extract onto a sheet as a breathable support used in the present invention, any method may be used so long as the ginkgo extract can be coated on a surface of the breathable support as uniform as possible. There may be exemplified a method for supporting a ginkgo extract onto a breathable support by applying the ginkgo extract as a solution or dispersion to the above-mentioned breathable support by any process such as coating, impregnating or spraying, and then removing the solvent or dispersing medium by drying or the like. As a solvent for the solution or dispersion, there may be mentioned various kinds of solvents to be used for the extraction of the ginkgo extract. Furthermore, there may be also mentioned a method for supporting the ginkgo extract by kneading the extract into a resin or metal which is a base material of the support. In addition to the above-mentioned methods, there may be also mentioned a method for adding and supporting the ginkgo extract in the course of sheeting a base fiber as in the adding in a wet papermaking process.

Although the fixation of the ginkgo extract itself to a support is firm, it is one of the preferred methods to use a little amount of binder (adhesive) in the range of not inhibiting the effect of the ginkgo extract when making the fixation of the ginkgo extract to the support more firm or when improving the appearance of the product by using the other components in combination or a coloring agent. Specific examples of hydrophilic binders are as follows. A water soluble binder includes, for example, polyvinyl alcohol, and starch. Also, a water dispersible binder includes, for example, poly (meth)acrylic acid esters, polyvinyl acetate, polyvinyl chloride, and styrene-butadiene latex.
Furthermore and optionally, in order to make the supporting of the ginkgo extract onto the support more stable, to keep the effects including allergen reduction constant for a more prolonged time, and to relief the odor of the ginkgo extract, porous powders (for example, powders of activated carbon or zeolite, or the like) may be contained in the breathable support.

Also, a material obtained by combining these sheets in which the ginkgo extract is supported thereon with the other sheets by laminating process, etc. is one of the preferred materials as a support in a plane shape.

As the other shape of the breathable support used in the present invention, there may be mentioned a solid shape such as a honeycomb shape, a corrugated shape and a pleated shape. As a material for constituting the support, there may be mentioned a plastic sheet having a poor breathing property such as polyolefin and polyester, a metallic board such as aluminum and copper, and a cardboard in addition to the one obtained by making the above-mentioned sheet-like material into a solid shape.

In addition to a method for applying the ginkgo extract to the support in a step before making the support into a solid shape, a method for spraying the ginkgo extract to the support in a step after making the support into the solid shape is also preferred.

Incidentally and optionally, for the purpose of adding the other properties to the air filter without departing from the spirit of the present invention, there may be used a support to which are newly applied various kinds of functions such as deodorizing, enhanced antibacterial activity, mold proofing, enhanced antiviral activity, insect proofing, insecticidal efficacy, odor eliminating, fragrance, thermosensing property, heat-retention, heat-accumulation, thermal storage, heat generation, heat absorption, waterproofing, water resistance, water-repellent property, hydrophobicity, hydrophilicity, dehumidification performance, moisture conditioning, moisture absorption, oil-repellent property, lipophilicity, adsorption of oil , and transpiration or sustained release of water or volatile chemicals.

In the present invention, it is favorable to add at least one function of deodorizing function, dust removal function, anti-microorganism function, fragrance-adding function and negative ions-adding function to the breathable support of the air filter as mentioned above in addition to the allergen inactivation activity.

By attaching the air filter of the present invention to an air processing device having a forced supply and exhaust property due to a machine, for example, an air-conditioning equipment, an air cleaner, a cleaner, a dehumidifier, a drier, a humidifier, an air fun, a fan and a heat exchanger, and then using the device, some preferred effects such as allergen reduction can be obtained. Alternately, the air filter of the present invention may be used for an inlet of the outdoor airflow (such as vent and window) for the natural supply and exhaust. In the case of an air cleaner and the like, a desired function such as deodorizing and dust removing may be obtained by using two or more filters having the respective functions, respectively, in combination.

### [Example]

In the following, the present invention will be specifically explained by referring to Examples.

### (Extraction of the ginkgo extract of April)

To 1 kg of dried ginkgo leaves (which was obtained by drying flesh leaves of April) was added 8 kg of mixed solution of ethanol and purified water (volume ratio = 1:1), and the mixture was stirred and extracted at 20-40°C for 7 hours. The solid was filtered off, and then the filtrate was concentrated under reduced pressure at 60°C or less until the whole volume of the filtrate became one tenth of the original volume. To the concentrate was added 0.5 kg of ethyl acetate and the resulting mixture was stirred sufficiently, and then, the mixture was subjected to standing and separation to give a lower layer. The lower layer was concentrated under reduced pressure to give a brown viscous material. The viscous material was dissolved in about 30 times amount of mixed solution of propylene glycol and purified water (volume ratio = 8:2), and a minor amount of insoluble material was filtered off by paper filter to give 8 g of ginkgo leaf extract solution. 1 g of this product corresponds to 0.1 g of dried ginkgo leaf extract.
In the case where this product is used in the respective Examples mentioned below, the above-mentioned operations were repeated until desired amount of the product was obtained.

### (Examples 1 to 7)

40 g/m² of polyester spun bond nonwoven fabric was used as a breathable support. The above-mentioned ginkgo leaf extract solution was uniformly coated to the nonwoven fabric by a spray method, and the nonwoven fabric was dried at 120°C to prepare the air filters of Examples. Incidentally, with respect to the amounts of the ginkgo leaf extract to be coated, 7 standards as a content of the dried ginkgo leaf extract were set as follows: 1 g/m² (Example 1), 5 g/m² (Example 2), 10 g/m² (Example 3), 15 g/m² (Example 4), 0.4 g/m² (Example 5), 0.04 g/m² (Example 6) and 0.004 g/m² (Example 7). Incidentally, when the respective amounts to be coated are converted to the contents of flavonol glycosides, they were 0.01 g/m² (Example 1), 0.05 g/m² (Example 2), 0.10 g/m² (Example 3), 0.15 g/m² (Example 4), 0.004 g/m² (Example 5), 0.0004 g/m² (Example 6) and 0.00004 g/m² (Example 7), respectively. Also, the respective flavonol glycosides were contained in the extract in an amount of 650% by weight based on terpene lactones.

### (Extraction of the ginkgo leaf extract from leaves of May)

Using the ginkgo leaf of May after drying, 8 g of the ginkgo leaf extract solution was obtained according to the similar method to the extraction method of the ginkgo leaf extract of April as mentioned above. 1 g of this product corresponds to 0.1 g of dried ginkgo leaf extract.
In the case where these products are used in the respective Examples mentioned below, the above-mentioned operations were repeated until desired amount of the product was obtained.

### (Examples 8 to 12)

Using the ginkgo leaf extract solution obtained from ginkgo leaves of May as mentioned above, air filters were obtained in the same manner as Examples 1 to 7. Incidentally, with respect to the amounts of the ginkgo leaf extract to be coated, 5 standards as a content of the dried ginkgo leaf extract were set as follows: 1 g/m² (Example 8), 5 g/m² (Example 9), 10 g/m² (Example 10), 20 g/m² (Example 11) and 0.5 g/m² (Example 12). Incidentally, when the respective amounts to be coated are converted to the contents of flavonol glycosides, they were 0.006 g/m² (Example 8), 0.03 g/m² (Example 9), 0.06 g/m² (Example 10), 0.12 g/m² (Example 11) and 0.003 g/m² (Example 12), respectively. Also, the respective flavonol glycosides were contained in the extract in an amount of 500% by weight based on terpene lactones.

### (Extraction of the ginkgo leaf extract from leaves of June)

Using the ginkgo leaf of June after drying, 8 g of the ginkgo leaf extract solution was obtained according to the similar method to the extraction method of the ginkgo leaf extract of April as mentioned above. 1 g of this product corresponds to 0.1 g of dried ginkgo leaf extract.
In the case where these products are used in the respective Examples mentioned below, the above-mentioned operations were repeated until desired amount of the product was obtained.

### (Examples 13 to 16)

Using the ginkgo leaf extract solution obtained from ginkgo leaves of June as mentioned above, air filters were obtained in the same manner as Examples 1 to 12. Incidentally, with respect to the amounts of the ginkgo leaf extract to be coated, 4 standards as a content of the dried ginkgo leaf extract were set as follows: 1 g/m² (Example 13), 5 g/m² (Example 14), 10 g/m² (Example 15) and 30 g/m² (Example 16). Incidentally, when the respective amounts to be coated are converted to the contents of flavonol glycosides, they were 0.004 g/m² (Example 13), 0.02 g/m² (Example 14), 0.04 g/m² (Example 15) and 0.12 g/m² (Example 16), respectively. Also, the respective flavonol glycosides were contained in the extract in an amount of 300% by weight based on terpene lactones.

### (Comparative Example 1)

The polyester spun bond nonwovon facric of Example was set as a filter of Comparative Example 1.

### (Comparative Examples 2 and 3)

Filters of Comparative Examples 2 and 3 were prepared in the same manner as Examples 2 and 3 except for using commercially available catechin extract (manufactured by ITO EN, LTD., TEAFRAN (tradename)) extracted from tea leaves instead of the ginkgo leaf extract solution used in Examples 2 and 3. (Extract contents of Examples 2 and 3 correspond to extract contents of Comparative Examples 2 and 3, respectively.)

The air filters of Examples 1 to 16 and the filters of Comparative Examples 1 to 3 as mentioned above were evaluated according to the following performance tests, and the results were shown in Table 1. Incidentally, the respective tests as mentioned below were carried out in the condition at 25°C and 50% RH (relative humidity).

### [Allergen inactivating test]

### 1. Preparations of reagents

### 1-1. Preparation of allergen solution

Purified tick allergen, rDerf2 (manufactured by SEIKAGAKU CORPORATION) was dissolved in PBS(-) to prepare 500 ng/ml of test allergen solution.

### 1-2. Preparation of antibody solution

Anti-rDerf2 monoclonal antibody 15E11 (manufactured by SEIKAGAKU CORPORATION) was diluted with PBS(-) to make a concentration of 2 µg/ml.

### 1-3. Preparation of labeled antibody solution

Horseradish peroxidase-labeled anti-rDerf2 monoclonal antibody 13A4PO (manufactured by SEIKAGAKU CORPORATION) was diluted 5000-fold with PBS-T. PBS-T was prepared by dissolving 0.5 g of Tween 20 in 1000 ml of PBS(-).

### 1-4. Preparation of 1%-BSA-PBS(-)

0.2 g of BSA was dissolved in 20 ml of PBS(-) to prepare 1%-BSA-PBS(-).

### 1-5. Preparation of 0.3 mg/ml-ABTS (substrate solution)

3 mg of ABTS was dissolved in 10 ml of 0.3 M-Citrate buffer (pH 4.0), and to the mixture was added 10 µl of 30% hydrogen peroxide to prepare 0.3 mg/ml-ABTS (substrate solution).

### 2. Test method

This test method is a test for confirming the matter whether the allergen trapped on the air filter is inactivated or not.

### 2-1. Summary

Air filters obtained from Examples 1 to 16 and Comparative Examples 1 to 3 were cut in a size of 2 mm x 4 mm to prepare specimens, respectively, and the specimens were tested respectively. The specimen was put on the bottom of 48-well plate. 300 µl of allergen solution (500 ng/ml) was added to each well and the plate was stood at room temperature for 2 hours. After standing, 50 µl of the solution was taken, and the concentration of allergen present in the solution was determined by sandwich ELISA method. The detail of the sandwich ELISA method is as follows.

### 2-2. Sandwich ELISA method

(1) 50 µl of coating solution (antibody solution, 2 µg/ml) was added to each well of ELISA plate, and the plate was stood at 4°C overnight.
(2) The coating solution was removed from the well, and the well was washed three times with each 300 µl of PBS(-). Subsequently, to the well was added 200 µl of 1%-BSA-PBS(-), and the plate was stood at room temperature for 1 hour.
(3) The ELISA plate was washed three times with each 300 µl of PBS-T. Subsequently, 50 µl of allergen solution which had been contacted with the specimen for 2 hours was taken and added to the each well of the plate, and the plate was stood at room temperature for 2 hours.
(4) The ELISA plate was washed three times with each 300 µl of PBS-T, and then, 50 µl of labeled antibody solution was added to the each well of the plate. The plate was stood at room temperature for 2 hours.
(5) The ELISA plate was washed three times with each 300 µl of PBS-T. Subsequently, 100 µl of 0.3 mg/ml-ABTS was added to the each well of the plate to occur coloring at room temperature, and after the reaction for 20 to 30 minutes, the plate was subjected to mixing. Absorbance at 405 nm was measured using a microplate leader. (6) Concentration (A ng/ml) of the purified tick allergen rDerf2 in the allergen solution which had been contacted with the specimen was calculated from the absorbance. By comparing the calculated value with the concentration (500 ng/ml) of purified tick allergen rDer2 in the initial allergen solution, an allergen removal ratio (%; 100x(500-A)/500) was calculated.

### [Virus inactivation test]

Air filters obtained from Examples 1 to 16 and Comparative Examples 1 to 3 were cut in a size of 3 cm x 3 cm to prepare specimens, respectively, and the specimens were tested respectively. To the specimens were added dropwise a suspension of influenza virus, and the specimens were preserved at room temperature, and after 24 hours, viral infectivities were measured. The detail is as follows.

### 1. Test virus

Influenza virus type A (H1N1) was used.

### 2. Used cells

MDCK (NBL-2) cell ATCC CCL-34 strains (manufactured by Dainippon Pharma Co., Ltd.) were used.

### 3. Used medium

### 3-1. Cell growth medium

Medium obtained by adding 10% fetal bovine serum to Eagle MEM (containing 0.06 mg/ml of kanamycin) was used.

### 3-2. Cell maintenance medium

Medium having a composition of 1000 ml of Eagle MEM, 34 ml of 10% aqueous sodium hydrogen carbonate solution, 9.8 ml of L-glutamine (30 g/l), 30 ml of MEM vitamin solution 100x, 20 ml of 10%-albumin, and 2 ml of trypsin (5 mg/ml) was used.

### 4. Preparation of virus suspension

### 4-1. Cell culture

MDCK cells were monolayer-cultured in a cell culture flask using the cell growth medium.

### 4-2. Virus inoculation

After the monolayer culture, the cell growth medium was removed from the inside of the flask, and the test virus was inoculated into the flask. Next, the cell maintenance medium was added to the flask and the flask was cultured inside a carbon dioxide incubator (carbon dioxide concentration: 5%) at 37°C for 2 to 5 days.

### 4-3. Preparation of virus suspension

After the culture, cell morphology was observed using an inverted phase contrast microscope. It was confirmed that morphology change (cytopathic effect) was occurred to the cells. Next, the culture medium was centrifuged (3000 rpm x 10 minutes), and the resulting supernatant fluid was referred to as a virus suspension.

### 5. Preparation of a sample

Air filters obtained from Examples 1 to 16 and Comparative Examples 1 to 3 were cut in a size of 3 cm x 3 cm to prepare specimens, respectively, and the specimens were moist heat sterilized (121°C x 15 minutes) to prepare samples.

### 6. Test operation

0.2 ml of the virus suspension was added dropwise to the sample, and the sample was preserved at room temperature. Also, a control test was conducted in the same manner as the above except for using a plastic plate as a control sample.

### 7. Washout of virus

After the preservation for 24 hours, virus suspension in the sample was washout with 2 ml of cell maintenance medium.

### 8. Measurement of viral infectivity

MDCK cells were monolayer-cultured in a tissue culture microplate (96-well) using the cell growth medium, and then, the cell growth medium was removed from the wells and 0.1 ml of cell maintenance medium was added to the each well of the microplate. Next, the washout liquid and a diluted solution thereof were inoculated into 4 wells, respectively, in an amount of 0.1 ml per well, and the cells were cultured inside a carbon dioxide incubator (carbon dioxide concentration: 5%) at 37°C for 4 to 7 days. After the culture, presence or absence of cell morphology change (cytopathic effect) was observed using an inverted phase contrast microscope. 50% tissue culture infection dose (TCID₅₀) was calculated by Reed-Muench method, and it was converted to viral infectivity per 1 ml of the washout liquid. Logarithm value of TCID₅₀ per 1 ml (log TCID₅₀/ml) of the washout liquid immediately after the virus inoculation (control) was compared with that of the washout liquid 24 hours after the preservation (samples), and the compared value was set as an indicator of virus inactivation activity. Incidentally, log TCID₅₀/ml < 1.5 was set as a detection limit.

### [Antibacterial activity test]

According to JIS Z 2801:2000 "Antimicrobial products - Test for antimicrobial activity and efficacy" 5.2 Test Methods for Plastic Products, etc., antibacterial activity tests for the respective specimens obtained from air filters of Examples 1 to 16 and Comparative Examples 1 to 3 were performed. As a bacterial species for the test, E. coli and staphylococcus aureus were used. The antibacterial activity value was set as an indicator of the antibacterial activity.

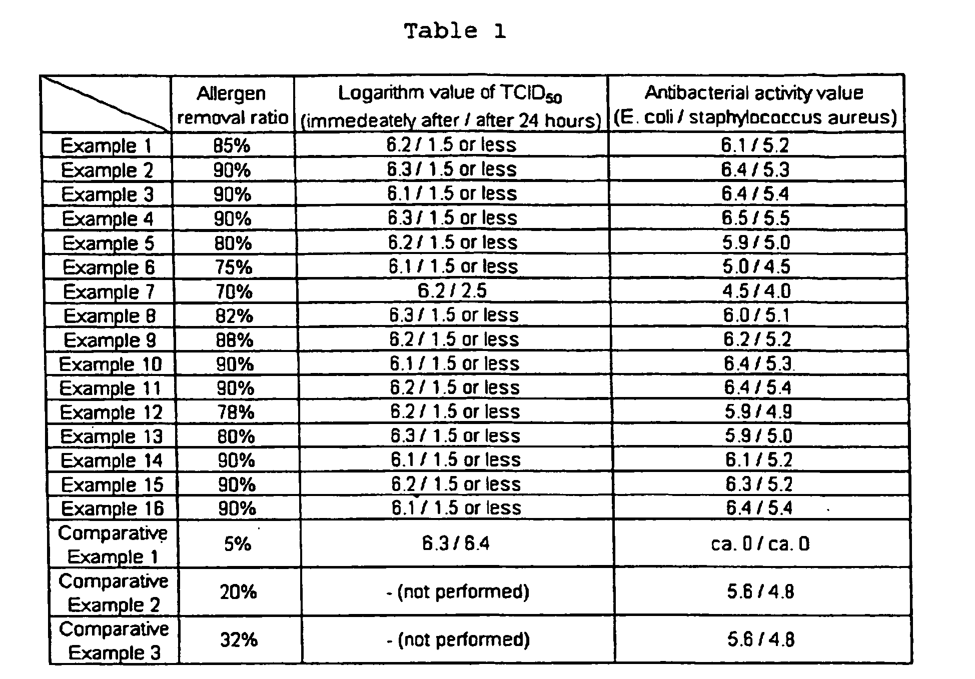

### [Actual use test performed by attaching the air filter to an air cleaner]

As a measurement of allergen inactivation effect due to an anti-allergen filter, filters obtained from Examples 1 to 3 and Comparative Example 1 were attached to the external air inlets of air conditioners, respectively, and the air conditioners were run about 3 hours a day in an air blasting condition for 7 days. By using a cleaner to which a dust sampler (manufactured by SHINTO FINE COMPANY, LIMITED) and MITEY-FELT (manufactured by SHINTO FINE COMPANY, LIMITED) were attached, dusts trapped on the attached filter were aspirated to the each MITEY-FELT. The felt into which the dusts were taken was put in a plastic-film bag with a zipper. To the bag was added 10 ml of phosphate buffer (pH 7.0, containing 15% by weight of bovine serum albumin), and the bag was kneaded sufficiently to extract a tick allergen. For the determination of the amount of the tick allergen, MITEY CHECKER (manufactured by SHINTO FINE COMPANY, LIMITED) which is a rapid test kit for indoor dust tick was used. Results of the tick allergen determination were shown in the following.

**Table 2**

| | Determination results |
|---|---|
| Example 1 | - |
| Example 2 | - |
| Example 3 | - |
| Comparative Example 1 | ++ |

| | |
|---|---|
| In the table, criteria for the amount of the tick allergen are as follows: ++: Dark and clear line. +: One can clearly understand it is a line. +-: One can understand it is lightly coloring -: Not coloring at all. | |

As mentioned above, it was confirmed that the anti-allergen filter used in the present invention can reduce the allergen trapped on the filter.

It was demonstrated that the air filter used in the present invention is a multifunctional air filter not only having an anti-allergen activity but also having an antiviral activity and an antibacterial activity, and, by applying the air filter to an air processing device (an air cleaner in the above example), preferred environment can be provided. In addition, there is no specific problem such as an influence on respiratory organ of human body, etc., or odor.

On the other hand, it was confirmed that a catechin extracted from tea leaves has a 3-hydroxy flavan structure which has a different structure from the flavonol glycosides in the ginkgo leaf extract, and the air filter obtained by using the tea leaf extract containing the catechin has a sufficient antibacterial activity but has an insufficient anti-allergen activity. It may be assumed that some components specific for the ginkgo extract which is explained in detail in the present specification act extremely effectively, and as a result, the above-mentioned difference was occurred.

Incidentally, the antiviral activity and antibacterial activity of the air filter obtained by the present invention are exhibited at least on the filter. Thus, it is easily expected that an air improved with respect to virus and bacteria by the air processing device, etc., to which the filter is attached can be supplied inside the room.

### [Industrial applicability]

The air filter used in the present invention can inactivate an allergen trapped on the filter. Furthermore, the air filter of the present invention can be applied to an air filter of various kinds of air processing devices such as an air conditioner, an air cleaner, a cleaner, dehumidifier, a drier, a humidifier, an air fun and a heat exchanger since it also has an antiviral activity and an antibacterial activity. In addition, it may be also applied to a humidification element of the humidifier, sterilizing agent for plumbing, or mask, wet wiper and floor wiper.

## Claims

1. Use of an air filter comprising a breathable support with a ginkgo extract contained therein, said breathable support having a plane shape for inactivating an allergen trapped on the air filter wherein the application is an air processing device.

2. The use according to claim 1, wherein the air processing device is an air cleaner.

3. The use according to claim 1 or 2, wherein the ginkgo extract is obtained by extraction of ginkgo leaves with a solvent selected from a group consisting of water, ethyl alcohol, acetone, ethyl ether, propylene glycol, butylene glycol, glycerin and acetic acid.

4. The use according to any one of claims 1 to 3, wherein the breathable support is fabricated into a three-dimensional shape.

5. The use according to any one of claims 1 to 4, wherein the amount of the ginkgo extract is 0.1 g/m² to 10.0 g/m² based on the area of the breathable support.

6. The use according to claim 5, wherein the amount of the ginkgo extract is 0.5 g/m² to 5.0 g/m² based on the area of the breathable support.

7. The use according to any one of claims 1 to 6, wherein the ginkgo extract contains flavonol glycosides and terpene lactones.

8. The use according to claim 7, wherein the amount of the flavonol glycosides is 300% by weight to 1500% by weight based on the amount of the terpene lactones.

9. The use according to claim 7, wherein the amount of the flavonol glycosides is 0.0004 g/m² to 0.15 g/m² based on the area of the breathable support.

## Patentansprüche

1. Verwendung eines Luftfilters, umfassend einen atmungsaktiven Träger mit einem darin enthaltenen Ginkgo-Extrakt, wobei der atmungsaktive Träger eine planare Gestalt hat, zur Inaktivierung eines auf dem Luftfilter gefangenen Allergens, wobei die Vorrichtung eine Luftaufbereitungsanlage ist.

2. Verwendung gemäß Anspruch 1, wobei die Luftaufbereitungsanlage ein Luftreiniger ist.

3. Verwendung gemäß Anspruch 1 oder 2, wobei der Ginkgo-Extrakt erhalten wird durch Extraktion von Ginkgoblättern mit einem Lösungsmittel, ausgewählt aus der Gruppe, bestehend aus Wasser, Ethylalkohol, Aceton, Ethylether, Propylenglycol, Butylenglycol, Glycerin und Essigsäure.

4. Verwendung gemäß einem beliebigen der Ansprüche 1 bis 3, wobei der atmungsaktive Träger als dreidimensionale Form hergestellt ist.

5. Verwendung gemäß einem beliebigen der Ansprüche 1 bis 4, wobei die Menge an Ginkgo-Extrakt 0,1 g/m² bis 10,0 g/m² ist, bezogen auf die Fläche des atmungsaktiven Trägers.

6. Verwendung gemäß Anspruch 5, wobei die Menge an Ginkgo-Extrakt 0,5 g/m² bis 5,0 g/m² ist, bezogen auf die Fläche des atmungsaktiven Trägers.

7. Verwendung gemäß einem beliebigen der Ansprüche 1 bis 6, wobei der Ginkgo-Extrakt Flavonolglycoside und Terpenlactone enthält.

8. Verwendung gemäß Anspruch 7, wobei die Menge an Flavonolglycosiden 300 Gew.-% bis 1500 Gew.-% ist, bezogen auf die Menge an Terpenlactonen.

9. Verwendung gemäß Anspruch 7, wobei die Menge an Flavonolglycosiden 0,0004 g/m² bis 0,15 g/m² ist, bezogen auf die Fläche des atmungsaktiven Trägers.

## Revendications

1. Utilisation d'un filtre à air comprenant un support respirant avec un extrait de Ginkgo contenu dans celui-ci, ledit support respirant ayant une forme plane, permettant d'inactiver un allergène piégé sur le filtre à air, dans laquelle l'application est un dispositif de traitement de l'air.

2. Utilisation selon la revendication 1, dans laquelle le dispositif de traitement de l'air est un épurateur d'air.

3. Utilisation selon la revendication 1 ou 2, dans laquelle l'extrait de Ginkgo est obtenu par extraction de feuilles de Ginkgo avec un solvant choisi dans un groupe constitué par l'eau, l'alcool éthylique, l'acétone, l'éthyl éther, le propylène glycol, le butylène glycol, la glycérine et l'acide acétique.

4. Utilisation selon l'une quelconque des revendications 1 à 3, dans laquelle le support respirant est fabriqué dans une forme tridimensionnelle.

5. Utilisation selon l'une quelconque des revendications 1 à 4, dans laquelle la quantité d'extrait de Ginkgo est de 0,1 g/m² à 10,0 g/m² par rapport à l'aire du support respirant.

6. Utilisation selon la revendication 5, dans laquelle la quantité d'extrait de Ginkgo est de 0,5 g/m² à 5,0 g/m² par rapport à l'aire du support respirant.

7. Utilisation selon l'une quelconque des revendications 1 à 6, dans laquelle l'extrait de Ginkgo contient des glycosides de flavonol et des lactones de terpène.

8. Utilisation selon la revendication 7, dans laquelle la quantité de glycosides de flavonol est de 300 % en poids à 1 500 % en poids par rapport à la quantité des lactones de terpène.

9. Utilisation selon la revendication 7, dans laquelle la quantité de glycosides de flavonol est de 0,0004 g/m² à 0,15 g/m² par rapport à l'aire du support respirant.
